# EUROPEAN PATENT APPLICATION

(11) **EP 3 679 917 A1**
(43) Date of publication of application: **15.07.2020**
(21) Application number: 19191731.9
(22) Date of filing: 14.08.2019
(51) Int. Cl.: A61K 8/02, A61Q 5/02

(54) **A SOLID SHAMPOO AND PREPARATION METHOD THEREOF**

(30) Priority: 08.01.2019 CN 201910015354
(71) Applicant: Ming Fai Innovative Skin Care Lab Limited, San Hop Lane, Tuen Mun N.T (HK)
(72) Inventor: CHAN, Yick Ning, San Hop Lane, Tuen Mun (HK); LIN, Hemei, Longgang, Shenzhen, 518100 (CN); CHAN, Chun Kit Frankie, San Hop Lane, Tuen Mun (HK)
(74) Representative: Isern Patentes y Marcas S.L.

(57) **Abstract**

The present invention provides a solid shampoo, including the following components in percentage by weight: sodium cocoyl isethionate 50-80%, first surfactant 1-10%, second surfactant 0.5-20%, polyhydric alcohol humectant 1-20% and preservative 0.1-2%; The first surfactant comprises one or two of the following: sodium lauryl sulfoacetate and disodium laureth sulfosuccinate. The second surfactant comprises one or more of the following: coco-glucoside, cocamidopropyl betaine and disodium lauroamphodiacetate. The solid shampoo is in the form of solid structure. The solid shampoo is gentle and non-irritating. The solid shampoo is free of irritating ingredients such as silicon oil. Recyclable or biodegradable paper packaging can be used preferably to reduce energy consumption. The present invention also provides a method for preparing the solid shampoo.

## Description

### TECHNICAL FIELD

The invention relates to the field of cosmetics, in particular to a kind of solid shampoo and its preparation method.

### BACKGROUND

At present, there are various shampoo products for hair cleaning but most of them are in the form of liquid. It is inconvenient for the user with specific propose such as business trip to carry and use the liquid shampoo. By increasing the usage of plastic containers, it causes serious pollution of the environment. Recently, Mintel released a report, pointing out the controversy caused by the harmful effect by large amount of packaging residues of food and beverage, thus the responsibility of cosmetics and personal care industry on reducing plastic packaging by following the trend. The report also predicts that plastic container in bathroom will not be allowed to use in the near future in hotel industry.

Apart from the packaging, some shampoo products contain silicone oil, harsh surfactants eliciting irritant reaction and other chemical components, causing side effects including hair damage and skin irritation by long term use. For example, in order to enhance the smoothness of the hair, a silicone oil component such as octacyclotetrasiloxane (D4) or decamethylcyclopentasiloxane (D5) is added to the shampoo. It has been reported that the European Chemicals Agency (ECHA) found that D4 and D5 are persistent, bioaccumulative, toxic, especially highly persistent and bioaccumulative in the environment, so they are in REACH Annex XIII.

With the advancement of society, people have higher expectation on the shampoo products; therefore, it is necessary to develop a mild, non-irritating, healthy and environmentally friendly solid shampoo.

### SUMMARY

In view of the above, the present invention provides a solid shampoo and its preparation method, the solid shampoo is in a solid state and is mild and non-irritating as there is no irritating component such as silicone oil. Recyclable or biodegradable paper packaging for the solid shampoo can reduce energy consumption and protect health and environment.

First, the present invention provides a solid shampoo comprising the following components by percentage weight:
sodium cocoyl isethionate 50-80%, first surfactant 1-10%, second surfactant 0.5- 20%, polyhydric alcohol humectant 1-20% and preservative 0.1-2%; the first surfactant comprises one or two of sodium lauryl sulfoacetate and disodium laureth sulfosuccinate, the second surfactant comprises one or more of coco-glucoside, cocamidopropyl betaine and disodium lauroamphodiacetate.

Further, optionally, the weight percentage of the sodium cocoyl isethionate in the solid shampoo is 55-75%. For example, the sodium cocoyl isethionate in the solid shampoo is 50% by weight, or 55%, or 60%, or 65%, or 70%, or 75%, or 80%. In the present invention, the sodium cocoyl isethionate is a mild, high foaming anionic surfactant which has excellent foaming property and strong resistance to hard water. It is easy to rinse and biodegradable; in particular, the sodium cocoyl isethionate has excellent mildness to the skin and can impart a silky skin feel to the skin after use.

Wherein, the first surfactant may be, but not limited to, sodium lauryl sulfoacetate or disodium laureth sulfosuccinate. Preferably, the first surfactant comprises sodium lauryl sulfoacetate. Further, optionally, the weight percentage of the first surfactant in the solid shampoo is 2-8%. For example, the first surfactant is 1% by weight, or 2%, or 5%, or 8%, or 10%. In the present invention, the first surfactant is a surfactant having excellent foaming properties and low irritation. Wherein, the sodium lauryl sulfoacetate is good at decontamination, foaming, solubilization, emulsification and also has good lime soap dispersing power and hard water resistance. Meanwhile, sodium lauryl sulfoacetate is milder and can also be used in infant washing products.

Further, optionally, the second surfactant is 5-15% by weight in the solid shampoo. For example, the second surfactant is present in the solid shampoo in a weight percentage of 1%, or 5%, or 8%, or 10%, or 15%, or 20%. The second surfactant may be, but not limited to, coco-glucoside, or cocamidopropyl betaine, or disodium lauroamphodiacetate, or coco-glucoside and cocamidopropyl betaine, or coco-glucoside and disodium lauroamphodiacetate. Preferably, the second surfactant comprises coco-glucoside.

In the present invention, the second surfactant is a surfactant having good suds-stabilizing ability and low irritation, and is suitable in combining with an anionic surfactant. Among them, the coco-glucoside is a 100% plant-derived nonionic surfactant with excellent biodegradability, good suds-stabilizing ability, and can reduce the irritation caused by other surfactants.

Optionally, the solid shampoo further comprises 0.1-10% hair conditioning factor by weight including one or more of hair conditioning oil, polymeric conditioning agent and scalp nutrients.

Further, optionally, the hair conditioning factor is 2-8% by weight in the solid shampoo. For example, the hair conditioning factor is 0.1% by weight, or 0.5%, or 1%, or 5%, or 8%, or 10%.

The hair conditioning factor of the present invention can perform scalp and hair care from the inside out, so that the scalp can have a healthy living environment. It provides superior conditioning and gives glossy and soft hair.

Optionally, among the hair conditioning factor, the hair conditioning oil is 50-70% by weight; the polyhydric alcohol humectants is 10-25% by weight and scalp nutrients is 20-40% by weight.

Wherein, the hair conditioning oil comprises one or more of cetyl ethylhexanoate, octyldodecanol, simmondsia chinensis (jojoba) seed oil, argania spinosa kernel oil, olive oil, camellia oil and macadamia oil. The hair conditioning oil comprises of synthetic oil and plant oil, which can moisturize scalp and hair, as well as soften the hair and make hair glossy. Further, in the hair conditioning oil , the weight percentage of the emollients synthetic oil is 20-40%, and that of the plant oil is 60-80%.

Among them, the polymeric conditioning agent comprises one or more of polyquaternium-10, polyquaternium-47, polyquaternium-39, quaternium-80 and acrylamidopropyltrimonium chloride/acrylamide copolymer. The polymeric conditioning agent can well regulate the wet and dry combing properties of hair, reduce the electrostatic effect of hair, and enhance the suppleness of hair.

Wherein, the scalp nutrient comprises one or more of creatine, PCA-Na or panthenol. The scalp nutrients provide the desired nutrients to the scalp to maintain the scalp in a healthy state. PCA-Na is a naturally occurring substance in skin and is an amino acid derivative. PCA-Na can improve the softness of skin so the skin can be softer and more elastic.

Optionally, the solid shampoo further comprises 0.01 to 0.3% plant essential oil by weight. Further, optionally, the plant essential oil is 0.05-0.2% by weight in the solid shampoo. For example, the plant essential oil is 0.01% by weight, or 0.05%, or 0.1%, or 0.15%, or 0.2%, or 0.25%, or 0.3%.

Optionally, the plant essential oil comprises one or more of chamaemelum nobile (chamomile) oil, rosmarinus officinalis (rosemary) leaf oil, salvia officinalis (sage) oil, citrus oil and lavender oil.. The plant essential oil is fragrant and extracted from a natural plant. The plant essential oil can effectively reduce nervous tension to a certain extent and cause a pleasant feeling.

Further, optionally, the weight percentage of the polyhydric alcohol humectant in the solid shampoo is 5-15%. For example, the polyhydric alcohol humectant is 1% by weight, or 5%, or 8%, or 10%, or 15%. Optionally, the polyhydric alcohol humectant further comprises one or more of glycerin, butylene glycol and sorbitol. For example, the polyhydric alcohol humectant includes glycerin and butylene glycol; or glycerin and sorbitol; or any one of glycerin, butylene glycol, and sorbitol. The polyhydric alcohol humectant can moisturize hair and scalp, and stabilize the formulation system.

Further, optionally, the preservative is present in the solid shampoo in a weight percentage of 0.3-1.2%. For example, the weight percent of the preservative in the solid shampoo is 0.3%, or 0.8%, or 1.0%, or 1.2%, or 1.5%, or 2%. Optionally, the preservative comprises one or more of sodium benzoate, potassium sorbate, ethylhexylglycerin and caprylyl glycol. The preservatives are mild, wherein sodium benzoate and potassium sorbate are food-grade preservatives, and ethylhexyl glycerol and octyl glycol are polyol with antiseptic property as well as moisturizing effect.

Optionally, the pH range of the solid shampoo of the invention is 5.0 to 6.0. Further, optionally, the pH range of the solid shampoo is 5.5 to 6.0. The solid shampoo is generally weakly acidic, conforming to the pH range of healthy hair and scalp so it can protect hair and scalp. Wherein, the pH value of the solid shampoo refers to the pH value of an aqueous solution of the solid shampoo of the invention at a mass fraction of 1% at 25°C. The pH range of some existing solid shampoos on the market is generally 9.0-10.0 or even higher and highly alkaline soap bases are used so it may cause severe stimulation on hair, skin and eyes. Compared with the existing shampoos, the pH range of the solid shampoo of the invention is 5.5-6.0, and all the components have mild characteristics, so the solid shampoo of the invention can effectively reduce the stimulation on human skin. Because of the characteristics of mild and non-irritating to the skin, it is suitable for all kinds of people.

Alternatively, the packaging material of the solid shampoo can be, but is not limited to, paper. The solid shampoo of the present invention can be packed as, but is not limited to, a box, the package material of which can be plastic or paper. For the sake of environmental protection and sustainable development, the packaging material of solid shampoo is preferred to be paper other than plastic, but is not limited to paper. Compared to a plastic case, it can be more cost effective and energy efficient by using paper packaging. Further, compared to the conventional liquid shampoos, the solid shampoo can reduce the use of a large amount of packaging material and reduce the transporting capacity. The solid shampoo can also reduce usage of water, and has a wide range of applications.

The solid shampoo proposed by the first aspect of the invention adopts sodium cocoyl isethionate and other raw materials which are mild and non-irritating to skin. The solid shampoo is good at foaming, well-adjusted, easy to rinse and excellent in use. Compared to traditional toiletries, the solid shampoo does not contain parabens, mineral oil, silicone oil and other irritating substances. The pH range of the solid shampoo is 5.0-6.0 which is suitable for the pH environment for healthy scalp and hair so it can be used for a long-term and make hair soft, shiny and perform well in skin care and hair care. The solid shampoo of the invention can be used for shampooing 10-15 times in only 10 g. Compared to the traditional liquid shampoo, the solid shampoo of the invention is more economical and durable.

The solid shampoo is suitable for all kinds of people, and can widely used for dry and oily hair. It is a healthy and mild shampoo and skin care product without soap base, mineral oil and silicone oil.

The solid shampoo of the invention is white or milky white in colour. Optionally, according to the preference of customers, the color of the solid shampoo can be changed by selectively adding healthy and non-irritating natural pigments.

Secondly, a method for preparing a solid shampoo proposed by the first aspect of the invention is as below:
Weigh each component according to the proportion of the formula, put it into a stirring pot, and obtain a mixture after stirring;

Optionally, The mixture is subjected to a grinding treatment, and then the mixture after the grinding treatment is molded to obtain a solid shampoo.

Optionally, the custom molding process is as below: printing the mixture processed by the grinding process into a solid shampoo of a predetermined shape by a printer device. Optionally, the weighing components are placed in a stirring pot in turn, and after continuous stirring, the components are evenly mixed to obtain the mixture. Optionally, the grinding time is 10-60 minutes.

Optionally, the solid shampoo comprises the following components by percentage weight: sodium cocoyl isethionate 50-80%, first surfactant 1-10%, second surfactant 0.5-20%, polyhydric alcohol humectant 1-20% and preservative 0.1-2%; the first surfactant 1-10% comprises one or two of sodium lauryl sulfoacetate and disodium laureth sulfosuccinate, the second surfactant comprises one or more of coco-glucoside, cocamidopropyl betaine and disodium lauroamphodiacetate.

Optionally, the polyhydric alcohol humectant further comprises one or more of glycerin, butylene glycol and sorbitol, the preservative comprises one or more of sodium benzoate, potassium sorbate, ethylhexylglycerin and caprylyl glycol.

Optionally, the solid shampoo further comprises 0.1-10% by weight of hair conditioning factor including one or more of hair conditioning oil, polymeric conditioning agent and scalp nutrients.

Optionally, the hair conditioning oil comprises one or more of cetyl ethylhexanoate, octyldodecanol, simmondsia chinensis (jojoba) seed oil, argania spinosa kernel oil, olive oil, camellia oil and macadamia oil; the polymeric conditioning agent comprises one or more of polyquaternium-10, polyquaternium-47, polyquaternium-39, quaternium-80 and acrylamidopropyltrimonium chloride/acrylamide copolymer; the scalp nutrient comprises one or more of creatine, PCA-Na or panthenol.

Optionally, the solid shampoo further comprises 0.01-0.3% plant essential oil by weight including one or more of chamaemelum nobile (chamomile) oil, rosmarinus officinalis (rosemary) leaf oil, salvia officinalis (sage) oil, citrus oil and lavender oil.
Optionally, the pH range of solid shampoo is 5.0-6.0.

Regarding the solid shampoo proposed by the second aspect of the invention, the process of the preparation method is simple, no extra heating is needed and energy can be saved. No additional pH regulation is needed for the product as the pH range of the solid shampoo can be maintained between 5.0 and 6.0 by the reasonable proportion of raw materials. Different from traditional soaps, this solid shampoo is superior in hair conditioning and can promote scalp health. By adding plant essential oil with antibacterial effect, the function of the solid shampoo can be further improved.

For the sake of environmental protection and sustainable development, the solid shampoo of the invention adopts packaging materials as paper other than plastic, but not limited to paper. For example, the solid shampoo is packaged with recyclable or biodegradable paper, so as to reduce the plastic packaging of cleaning and care products thereby reducing the environment pollution and damage and achieving sustainable development. Further, the solid shampoo mentioned in the invention also has good biodegradability. Together with the biodegradable paper packaging, the solid shampoo of the invention can be energy saving, environmental friendly and greatly reduce the production cost.

The shape and size of the solid shampoo can be varied by presetting various shapes. For example, the cross section shape of the solid shampoo can be a circle, rectangle, triangle or polygon.

Third, the invention also provides a cleaning product, which includes a solid shampoo as proposed in part 1 of the invention. For example, the shampoo product may include, but is not limited to, the main ingredients of the solid shampoo.

The advantages of the invention will be explained in part of the following specification, part of which is clear according to the specification or can be realized through the embodiment of the invention.

### BRIEF DESCRIPTION OF THE DRAWINGS

In order to explain the content of the invention more clearly, the following shows the detail in combination with attached drawings and specific embodiment.

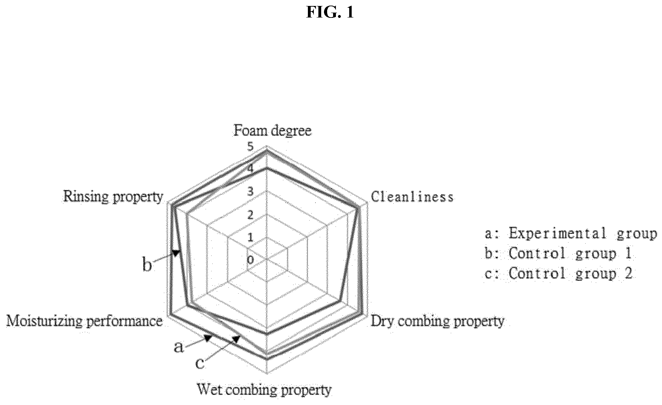

Fig. 1 is a comparison diagram of performance tests of different shampoos provided in one embodiment of the invention.

### DETAILED DESCRIPTION OF THE EMBODIMENTS

The following is the preferred embodiment of the invention. It should be pointed out that for technicians in the field, on the premise of not deviating the invention from the principle of embodiment, some improvements and retouching can be made, and these improvements and retouching are also regarded as the protection scope of the embodiment of the invention.

Embodiment of the present invention is further described as the following. Embodiment of the invention is not limited to the following specific embodiment. Without amendment in the claims, changes can be implemented appropriately. Unless there is any special instruction, the raw materials and other chemical reagents used in the embodiment of the present invention are all commercially available.

Embodiment 1 A preparation method for a solid shampoo, wherein the formulation is as follows:

| Raw Material | Material Proportion (%) |
|---|---|
| Sodium cocoyl isethionate | 55 |
| Sodium lauryl sulfoacetate | 8 |
| coco-glucoside | 15 |
| polyhydric alcohol humectant | 15 |
| Hair conditioning factor | 5.7 |
| Preservative | 1.2 |
| Plant essential oil | 0.1 |

### Preparation steps are as below:

Each component was weighed according to the proportion of the formula, and 55g sodium cocoyl isethionate was put into a stirring pot. 8g sodium lauryl sulfoacetate and 15g coco-glucoside were added slowly, and the three ingredients were mixed evenly. Then add 9g glycerin, 6g butylene glycol, 0.74g cetearyl ethylhexnoate, 2.96g camellia oil, 0.8g polymeric conditioning agent polyquaternium-39, 1.2g creatine, 0.8g caprylyl glycol, 0.4g ethylhexylglycerin, 0.1g chamaemelum nobile (chamomile) oil, and mix them evenly to get the mixture.

Grind the mixture evenly through the grinder for 20 minutes, put the mixture after grinding into the printer, and print them into the required shape through the printer to obtain the solid shampoo.

Embodiment 2 A preparation method for a solid shampoo, wherein the formulation is as follows:

| Raw Material | Material Proportion (%) |
|---|---|
| Sodium cocoyl isethionate | 67 |
| Sodium lauryl sulfoacetate | 7 |
| coco-glucoside | 5 |
| polyhydric alcohol humectant | 12.65 |
| Hair conditioning factor | 8 |
| Preservative | 0.3 |
| Plant essential oil | 0.05 |

### Preparation steps are as below:

Each component was weighed according to the proportion of the formula, and 67g sodium cocoyl isethionate was put into a stirring pot. 7g sodium lauryl sulfoacetate and 5g coco-glucoside were added slowly, and the three ingredients were mixed evenly. Then add 7.59g glycerin, 5.06g sorbitol, 2.24g cetearyl ethylhexnoate, 3.36g camellia oil, 1.2g quaternium-80, 1.2g PCA-Na, 0.3g sodium benzoate, 0.05g rosmarinus officinalis (rosemary) leaf oil, and mix them evenly to get the mixture.

Grind the mixture evenly through the grinder for 30 minutes, put the mixture after grinding into the printer, and print them into the required shape through the printer to obtain the solid shampoo.

Embodiment 3 A preparation method for a solid shampoo, wherein the formulation is as follows:

| Raw Material | Material Proportion (%) |
|---|---|
| Sodium cocoyl isethionate | 75 |
| Sodium lauryl sulfoacetate | 2 |
| coco-glucoside | 12 |
| polyhydric alcohol humectant | 5 |
| Hair conditioning factor | 4.8 |
| Preservative | 1 |
| Plant essential oil | 0.2 |

### Specific preparation steps include:

Each component was weighed according to the proportion of the formula, and 75g sodium cocoyl isethionate was put into a stirring pot. 2g sodium lauryl sulfoacetate and 12g coco-glucoside were added slowly, and the three ingredients were mixed evenly. Then add 3g butylene glycol, 2g sorbitol, 1.2g octyldodecanol, 1.8g macadamia oil, 0.8g polyquaternium-47, 1.0g panthenol, 0.5g caprylyl glycol, 0.3g sodium benzoate, 0.2g salvia officinalis (sage) oil, and mix them evenly to get the mixture.

Grind the mixture evenly through the grinder for 20 minutes, put the mixture after grinding into the printer, and print them into the required shape through the printer to obtain the solid shampoo.

Embodiment 4 A preparation method for a solid shampoo, wherein the formulation is as follows:

| Raw Material | Material Proportion (%) |
|---|---|
| Sodium cocoyl isethionate | 71 |
| Sodium lauryl sulfoacetate | 6 |
| coco-glucoside | 10 |
| polyhydric alcohol humectant | 10.2 |
| Hair conditioning factor | 2 |
| Preservative | 0.62 |
| Plant essential oil | 0.18 |

### Preparation steps are as below:

Each component was weighed according to the proportion of the formula, and 71g sodium cocoyl isethionate was put into a stirring pot. 6g sodium lauryl sulfoacetate and 10g coco-glucoside were added slowly, and the three ingredients were mixed evenly. Then add 2.04g glycerin, 8.16g sorbitol, 0.2g cetearyl ethylhexnoate, 0.2g octyldodecanol, 0.6g macadamia oil, 0.3g polyquaternium-10, 0.7g panthenol, 0.32g potassium sorbate, 0.3g sodium benzoate, 0.09g citrus oil, 0.09g lavender oil, and mix them evenly to get the mixture.

Grind the mixture evenly through the grinder for 20 minutes, put the mixture after grinding into the printer, and print them into the required shape through the printer to obtain the solid shampoo.

Embodiment 5 A preparation method for a solid shampoo, wherein the formulation is as follows:

| Raw Material | Material Proportion (%) |
|---|---|
| Sodium cocoyl isethionate | 72.5 |
| Sodium lauryl sulfoacetate | 4 |
| coco-glucoside | 8 |
| polyhydric alcohol humectant | 8.15 |
| Hair conditioning factor | 6.4 |
| Preservative | 0.8 |
| Plant essential oil | 0.15 |

### Preparation steps are as below:

Each component was weighed according to the proportion of the formula, and 72.5g sodium cocoyl isethionate was put into a stirring pot. 4g sodium lauryl sulfoacetate and 8g coco-glucoside were added slowly, and the three ingredients were mixed evenly. Then add 1.63g glycerin, 1.63g butylene glycol, 4.89g sorbitol, 1.04g octyldodecanol, 1.56g macadamia oil, 1.56g camellia oil, 1.0g acrylamidopropyltrimonium chloride/acrylamide copolymer, 1.24g PCA-Na, 0.5g caprylyl glycol, 0.3g potassium sorbate, 0.15g lavender oil, and mix them evenly to get the mixture.

Grind the mixture evenly through the grinder for 20 minutes, put the mixture after grinding into the printer, and print them into the required shape through the printer to obtain the solid shampoo.

Embodiment 6 A preparation method for a solid shampoo, wherein the formulation is as follows:

| Raw Material | Material Proportion (%) |
|---|---|
| Sodium cocoyl isethionate | 72.5 |
| Sodium lauryl sulfoacetate | 4 |
| coco-glucoside | 8 |
| polyhydric alcohol humectant | 8.15 |
| Hair conditioning factor | 6.4 |
| Preservative | 0.8 |
| Plant essential oil | 0.15 |

### Preparation steps are as below:

Each component was weighed according to the proportion of the formula, and 72.5g sodium cocoyl isethionate was put into a stirring pot. 4g disodium laureth sulfosuccinate and 8g cocamidopropyl betaine were added slowly, and the three ingredients were mixed evenly. Then add 1.63g glycerin, 1.63g butylene glycol, 4.89g sorbitol, 1.04g octyldodecanol, 1.56g macadamia oil, 1.56g camellia oil, 1.0g acrylamidopropyltrimonium chloride/acrylamide copolymer, 1.24g PCA-Na, 0.5g caprylyl glycol, 0.3g potassium sorbate, 0.15g citrus oil, and mix them evenly to get the mixture.

Grind the mixture evenly through the grinder for 25 minutes, put the mixture after grinding into the printer, and print them into the required shape through the printer to obtain the solid shampoo.

### Trial on effect of the embodiment and contrast test

The solid shampoo obtained from embodiment 5 of the invention is taken as the experimental group, and the solid shampoo of brand popular on web on the market and the liquid smooth shampoo of a brand with good reputation in the market are respectively taken as control group 1 and control group 2. A total of 75 women aged 25-35 years were selected and divided into 3 groups for the test. Among them, the number of women with long hair was with same proportion in each group. During the trial, hair was washed every two days in two weeks. The comparison of the results after use is shown in the table 1 (the following is scored in the form of a full score of 5, and the following results are obtained after averaging the total scores of all results) :

**Table 1: Testing data comparison table**

| Group | User Experience Evaluation | | | | | | Comprehensive Evaluation | | |
|---|---|---|---|---|---|---|---|---|---|
| | Foam degree | Cleanliness | Dry combing property | Wet combing property | Moisturizing performance | Rinsing property | Excellent | Good | Fine |
| Experimental group | 4.8 | 4.6 | 4.72 | 4.38 | 4.82 | 4.77 | √ | | |
| Control group 1 | 4.0 | 4.5 | 3.65 | 3.26 | 4.0 | 4.65 | | | √ |
| Control group 2 | 4.7 | 4.6 | 4.56 | 4.12 | 3.76 | 4.02 | | √ | |

From the test results in table 1 and figure 1 , it can be seen that the solid shampoo of the invention is far superior to other solid shampoo on the market in foam degree and cleanliness, especially in wet and dry combing properties. The solid shampoo in terms of moisturizing performance and rinsing property is also better than the smooth shampoo of current brand in market. From the comprehensive evaluation, it can be seen that the recognition of the solid shampoo by consumers is much higher than other products on the market.

The solid shampoo of the invention can be packaged with recyclable or biodegradable cardboard or paper, so as to reduce the use of plastics. In order to protect the environment, limiting the use of plastic and not polluting the environment can achieve sustainable development. The invention provides a new concept and a new direction for the development of cleaning products. It embodies the concept of environmental protection and sustainable development and has great application prospects. The solid shampoo provided by the embodiment of the invention is mild, non-irritating, healthy and safe, with high durability, and can be used repeatedly with an unit quantity. Compared with the existing shampoo products, the solid shampoo of the invention is more economical and practical, which is of great significance for prevalence in use of the solid shampoo.

The above disclosure is only a better embodiment of the invention, and of course cannot be used to limit the scope of the rights of the invention. Technicians in the field can understand all or part of the process of the above embodiment, and the equivalent changes made according to the claims of the invention still fall within the scope of the invention.

## Claims

1. A solid shampoo comprises the following components by percentage weight: sodium cocoyl isethionate 50-80%, first surfactant 1-10%, second surfactant 0.5- 20%, polyhydric alcohol humectant 1-20% and preservative 0.1-2%; the first surfactant comprises one or two of the following: sodium lauryl sulfoacetate and disodium laureth sulfosuccinate, the second surfactant comprises one or more of the following: coco-glucoside, cocamidopropyl betaine and disodium lauroamphodiacetate.

2. The solid shampoo according to claim 1, wherein the solid shampoo further comprises from 0.1 to 10% by weight of hair conditioning factor including one or more of hair conditioning oil, polymeric conditioning agent and scalp nutrients.

3. The solid shampoo according to claim 2, wherein the hair conditioning oil comprises one or more of cetyl ethylhexanoate, octyldodecanol, simmondsia chinensis (jojoba) seed oil, argania spinosa kernel oil, olive oil, camellia oil and macadamia oil; the polymeric conditioning agent comprises one or more of polyquaternium-10, polyquaternium-47, polyquaternium-39, quaternium-80 and acrylamidopropyltrimonium chloride/acrylamide copolymer; the scalp nutrient comprises one or more of creatine, PCA-Na or panthenol.

4. The solid shampoo according to claim 1, wherein the solid shampoo further comprises by weight percentage of 0.01-0.3% plant essential oil including one or more of chamaemelum nobile (chamomile) oil, rosmarinus officinalis (rosemary) leaf oil, salvia officinalis (sage) oil, citrus oil and lavender oil.

5. The solid shampoo according to claim 1, wherein the solid shampoo comprises polyhydric alcohol humectants including one or more of glycerin, butylene glycol and sorbitol.

6. The solid shampoo according to claim 1, wherein the solid shampoo comprises preservative including one or more of sodium benzoate, potassium sorbate, ethylhexylglycerin and caprylyl glycol.

7. The solid shampoo according to claim 1, wherein the pH range of the solid shampoo is 5.0-6.0.

8. The solid shampoo according to claim 1, wherein the solid shampoo uses recyclable or biodegradable paper packaging.

9. A preparation method for the solid shampoo according to any one of claims 1-8 is listed as below:
Weigh each component according to the proportion of the formula, put it into a stirring pot, and obtain a mixture after stirring;
The mixture is subjected to a grinding treatment, and then the mixture after the grinding treatment is molded to obtain a solid shampoo.

10. The preparation method according to claim 9, wherein the custom molding process is as below: printing the mixture processed by the grinding process into a solid shampoo of a predetermined shape by a printer device.
